# FASCICULE DE BREVET EUROPEEN

(11) **EP 0 373 986 B1**
(45) Date de publication et mention de la délivrance du brevet: **08.09.1993**
(21) Numéro de dépôt: 89403176.4
(22) Date de dépôt: 17.11.1989
(51) Int. Cl.: A61K 35/78, A61K 31/475

(54) **Composition antivirale et ses applications**
Virushemmende Zusammensetzung und ihre Anwendungen
Anti-virus composition and its uses

(30) Priorité: 02.12.1988 FR 8815845
(43) Date de publication de la demande: 20.06.1990
(73) Titulaire: ABRAXAS BIO LABS S.A., L-2121 Luxembourg-Kirchberg (LU)
(72) Inventeur: Beljanski, Mirko, F-75005 Paris (FR)
(74) Mandataire: Pinguet, André

(56) Documents cités:
- EP-A- 0 059 817
- FR-A- 2 450 607

## Description

Les infections virales posent aux médecins de multiples problèmes. Les agents pharmaceutiques capables de lutter efficacement et spécifiquement contre les virus sont en nombre plus que limité. En outre, ils provoquent généralement des effets secondaires indésirables. Les infections virales, non seulement détruisent les cellules hôtes, mais affectent aussi le fonctionnement de diverses protéines et enzymes. L'invasion virale favorise l'infection par d'autres agents pathogènes, d'autres virus ou bactéries, champignons... Ainsi, le virus de l'immunodéficience humaine (VIH) semble être la cause principale du syndrome du Sida chez les humains. Par la perte immunitaire qu'il engendre, ce virus ouvre la voie à d'autres virus (herpès simplex, cytomégalovirus, virus de l'hépatite B) et agents pathogènes qui envahissent l'organisme humain, créant des situations dangereuses. Le virus du Sida (VIN I et II) provoque la chute du nombre des lymphocytes, particulièrement des T4, T8 et des cellules B qui sont directement nécessaires lors de la lutte contre les agents pathogènes.

De nombeuses années de recherches intensives n'ont permis la mise au point que de peu de médicaments, dont aucun n'est dénué d'inconvénients. Le vaccin ou des médicaments plus spécifiques contre le Sida ne sont pas encore développés.

La présente invention repose sur une stratégie rationelle où, sans effets secondaires néfastes, la multiplication des virus, virus à ARN (Sida en particulier) et virus à ADN est inhibée tandis que le système immunitaire est stimulé. Cette stratégie qui possède de solides bases théoriques et pratiques, combine l'utilisation de quatre substances offrant des effets coopératifs à différents niveaux. L'association de ces substances est essentielle pour juguler l'infection virale, en particulier l'infection par le virus du Sida, car si chaque substance joue un rôle bien défini à un niveau biologique précis, aucune n'est suffisante. Il est important de souligner qu'il n'y a pas de compétition entre ces quatre différentes substances chez le patient atteint d'infection virale. Autrement dit, les effets bénéfiques qu'apporte chaque substance ne sont pas entravés par la présence des trois autres substances. Autre aspect important de l'invention, les molécules ne présentent pas d'effet secondaire toxique ou d'effet indésirable, ni séparément, ni lorsqu'elles sont associées.

L'utilisation combinée de ces quatre substances dans la lutte antivirale constitue la base de la présente invention. Elles sont : (1) la flavopéreirine (un alcaloïde de la classe des β-carbolines) et/ou son dérivé la dihydroflavopéreirine ; (2) la naringine et/ou la naringénine (flavanones) ; (3) les ARN amorceurs (ARN-fragments particuliers) ; (4) le Bioparyl, préparation particulière et standardisée obtenue à partir des feuilles dorées de l'arbre Gingko Biloba (voir brevet français n° 88 09738 du 19/07/88).

### Première et seconde substance

Les substances (1) et (2) sont groupées dans le présent exposé car elles possèdent de nombreuses propriétés communes bien que des différences importantes existent et qui seront précisées en détail plus loin.

Les substances (1) et (2) arrêtent la multiplication virale, virus à ARN ou virus à ADN, mais n'ont pas d'effet agressif vis-à-vis des ARN ou ADN des cellules normales. Leur action est sélective vis-à-vis des génomes non conformes, c'est-à-dire ceux des cellules virosées ou ceux des cellules cancéreuses. En outre chacune de ces substances bloque l'activité de la transcriptase inverse ainsi que l'activité d'une déoxynucléotidyl transférase terminale. L'une (1) franchit la barrière hématoencephalique, ce qui dans la lutte contre l'herpès et le Sida en particulier est essentielle, ces virus induisant des syndromes neurologiques sévères.

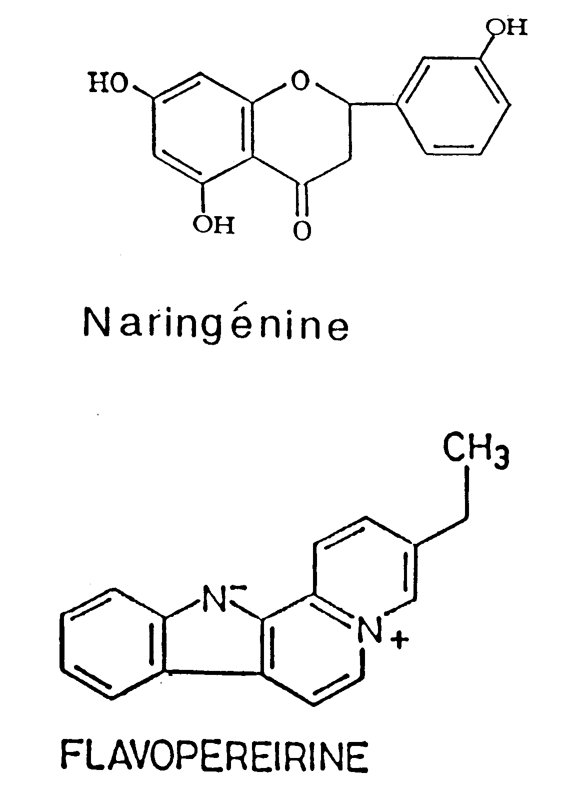

Ainsi l'emploi des substances (1) et (2) permet une action sélective dans les processus viraux et cancéreux, même au-delà de la barrière cérébrale, sans toxicité pour les cellules normales, et en particulier les cellules hématologiques qui sont très sensibles à la cytotoxicité des agents thérapeutiques et chimiques.

La substance (1), alcaloïde de la classe des β-carboline, est la flavopéreirine, qui agit in vivo contre le virus de l'influenza (virus à ARN), ainsi que contre les virus de l'herpès et du fibrome de Shope (virus à ADN). Certificat d'addition n° 79 05853 publié sous le n° 2 450 607 B.O.P.I. "Listes" n° 40 du 3 octobre 1980 (M. Beljanski et J. Bugiel). Elle peut en outre inhiber la multiplication du virus de la mosaïque du tabac (VMT) lors d'un bref contact avec ce virus. Brevet français n° 88 08434 du 23 juin 1988 (M. Beljanski).

La flavopéreirine agit au niveau des sites d'initiation de l'ADN des cellules cancéreuses ou au niveau de la transcription, franchit la barrière du cerveau de la souris, y séjourne environ 30 minutes, sauf si des cellules cancéreuses ou virosées sont présentes. Dans ce cas, elle se fixe à l'ADN de ces cellules, empêchant leur multiplication. Chez les humains, il en est de même.

Les propriétés de la flavopéreirine pour la destruction sélective des cellules cancéreuses et l'arrêt de la multiplication de certains virus sont décrites dans le brevet européen n° 0 059 817, accordé le 9 octobre 1985. La flavopéreirine fut isolée à partir des écorces de "Tariri Céliata Martius", une variété d'apocynaceae selon la méthode de H. Rapoport et Coll (J. American Chem. Soc. 80 : 1601-1608 (1958)).

La deuxième substance, une flavanone, est la naringine et/ou la naringénine, deux substances qui peuvent être utilisées de façon interchangeable dans la présente invention.

La référence à l'une ou à l'autre implique chacune séparément ou en combinaison. Lorsqu'on se réfère à l'une ou à l'autre, ceci inclut aussi les sels et esters acceptables sur le plan pharmaceutique, par exemple Sels de magnésium, sodium et naringenine acétylée.

La naringine et la naringénine possèdent certaines propriétés de la Flavopéreirine, mais quelques différences importantes existent : (1) ces deux substances ne semblent pas passer la barrière cérébrale. Contrairement à la flavopéreirine qui n'agit qu'au niveau de l'initiation, elles agissent au niveau des sites d'initiation et d'élongation des ADN des cellules cancéreuses. Elles inhibent in situ la mutiplication du VMT en solution. Différents virus (VIH, Herpès simplex, Epstein Barr ...) sont impliqués dans divers cancers humains, (sarcome de la bouche, de la peau, lymphome, sarcome de Kaposi, ...). L'association : flavopéreirine et naringine (naringénine) assure une parfaite inactivation des génomes viraux ou des génomes des cellules cancéreuses, ce qui permet une remarquable double-action contre les cellules virosées et/ou cancéreuses.

Le mode de préparation et les propriétés de la naringine et de la Naringénine ont été décrites dans le brevet français n° 88 08434 du 23 juin 1988 au nom de M. Beljanski.

### Troisième substance

Lors de la maladite induite par le virus HIV (Sida), une destruction dramatique des lymphocytes se produit. Or ces cellules sont essentielles dans la lutte immunologique contre tous les agents pathogènes. Les lymphocytes T4, T8 B diminuent spectaculairement chez la plupart des malades atteints du Sida. Les ARN amorceurs (désignés aussi R.L.B.) qui sont des ARN-fragments particuliers, stimulent de façon très sélective la génèse de toutes les cellules de la lignée blanche, c'est-à-dire les granulocytes et les lymphocytes. Mais, fait essentiel, seuls les leucocytes normaux et les plaquettes sanguines subissent un accroissement de leur génèse, accroissement qui cependant reste toujours dans des limites physiologiques. C'est en stimulant la replication de l'ADN des cellules hématopoietiques que les R.L.B., relativement résistants à l'action des ribonucléases, peuvent favoriser l'hématopoiese et activer les gènes des leucocytes (granulocytes et lymphocytes). Ils n'agissent que sur la génèse des cellules saines et leur différentiation. Ils augmentent de façon non négligeable le nombre de lymphocytes totaux dont différentes sous populations T ainsi que celui de lymphocytes B, contribuant ainsi à l'élimination des cellules pathologiques. Ils semblent également activer le système de réparation des cassures chromosomiques. Il semble plausible que les R.L.B. participent indirectement dans l'excision du génome HIV des cellules infectées.

Il est très important de souligner que sans action sur les cellules cancéreuses ou virosées, ils n'entravent aucunement l'action des substances antivirales.

Simples chaînes de ribonucléotides comportant environ 20 à 80 unités de rinucléotides (ou prédominent les séquences GA), les R.L.B. ont un rapport global de bases puriques/bases pyrimidiques (G + A/C + U) compris entre 1,0 et 2,5. Ils sont obtenus par scission enzymatique d'ARN selon le procédé décrit en détail dans le brevet USA n° 4 190 649 (1980). Voir aussi BELJANSKI et al. Cancer Treatment reports vol. n° 7-8, juillet-août 1983, pp. 611-619.

### Quatrième substance

La quatrième substance est tout à fait nécessaire pour compléter la stratégie thérapeutique, en ce sens que cancer ou maladie virale induisent des anomalies de certaines protéines enzymatiques. Il s'agit donc de réguler ces divers paramètres biologiques, soit qu'ils soient trop actifs ou trop peu actifs, soit encore qu'ils soient quantitativement modifiés par la pathologie. Sans l'effet régulateur biologique du produit, nommé Bioparyl, ces paramètres normalisent, en particulier les ribonucléases, enzymes dont les anomalies fonctionnelles participent activement et indirectement au processus pathologique. Le régulateur Bioparyl qui est totalement dénué d'effet toxique freine aussi de façon fort efficace l'accumulation tout à fait anormale de gamma-globulines lors de l'infection par le virus HIV.

L'effet du Bioparyl est progressif, conduisant à une légère répression d'une super-extension de certains gènes, responsables de la programmation des gamma-globulines. Ainsi, lors d'un fort excès d'immuno-globuline, IgG par exemple, un ou deux mois de traitement par le Bioparyl permet de ramener le taux de ces globulines vers des valeurs normales ainsi que l'activité des RNases.

Les propriétés du Bioparyl ne sont en rien entravées --bien au contraire-- par la présence des trois autres substances et l'état clinique s'en trouve rapidement amélioré.

Le Bioparyl est un extrait standardisé obtenu par extraction, hydrolise et purification d'extraits de feuilles dorées automnales de l'arbre Gingko Biloba. Le mode de préparation et les propriétés du Bioparyl ont été décrites dans le brevet d'invention n° 88 09738 du 19 juillet 1988, au nom de M. Beljanski.

### ADMINISTRATION ET DOSAGES

La combinaison des quatre substances utilisées dans la stratégie antivirale proposée peut être administrée à tous les animaux, mammifères en particulier, souffrant d'infection virale. Mais son intérêt principal est indiqué naturellement dans le traitement des humains. Trois des quatre substances peuvent être administrées séparément, groupées par deux ou par trois, mais l'invention requiert la présence simultanée de toutes aux bonnes doses, dans l'organisme à traiter. Les R.L.B. (3) seront donnés à part (voie perlinguale). Du fait de sa non-toxicité, le traitement peut être poursuivi pendant tout le temps nécessaire, voire des mois.

L'administration préconisée est la voie orale (sublinguale pour les R.L.B.) sous forme galénique habituelle : solution, tablettes, pilules gélules ou. Les voies parentérales, intraperitonéales, intraveineuses ou intramusculaires sont possibles (mais moins recommandées). De toute façon, qu'elles soient utilisées séparément ou ensemble, chacune des substances ou la combinaison de plusieurs sera normalement conditionnée à l'aide d'un support pharmaceutique convenable adapté à la voie d'administration.

Les dosages de chaque substance pourront varier selon l'atteinte virale du mammifere à traiter.

Pour la forme orale, des doses quotidiennes de 0,25 à 1 gr de flavopéreirine, de 0,5 à 1,5 gr de naringine (naringénine) et de 0,5 à 1 gr de Bioparyl peuvent être administrées (ou prévues pour l'administration). 10 à 40 milligrammes de R.L.B. seront retenus comme marge possible pour une administration sublinguale. Il sera préférable de réserver une dose journalière pour les R.L.B. et des prises fractionnées à plusieurs moments de la journée pour les autres produits.

Une fois choisi un conditionnement donné, la quantité de produit administré dépendra du choix du médecin prescripteur et de l'état d'avancement de la maladie. On peut envisager de disposer (médecin, pharmacien, vétérinaire) de produits en kit qui seront administrés soit ensemble, soit séparément.

Pour combattre le Sida, les deux antiviraux et le Bioparyl sont nécessaires, l'administration de R.L.B. restant à l'appréciation du thérapeute. Ceci dépend du taux des leucocytes.

Les séropositifs ne développant pas encore la maladie et, de ce fait, n'ayant pas ou très peu d'atteinte immunitaire, pourraient ne pas recevoir les R.L.B. tant que leur formule leucocytaire sera correcte.

### PREPARATION DES SUBSTANCES

I. Favopéreirine :
   La méthode d'obtention de cette substance à partir de l'écorce de "Pao Pereira" (tariri ciliata Martius) est celle décrite par H. Rapoport et coll. (J. American Chem. Soc. 80 ; 1601-1608 (1958)) : M. Beljanski et J. Bugiel, demande de certificat d'addition français n° 79 05853 du 7 mars 1979 et brevet européen n° 81 400 374.5 délivré le 10 septembre 1985, (0 059 817) Bulletin 82/37. La substance ainsi isolée est dépourvue d'effet toxique et sans effet secondaire, de même son dérivé dihydro.
II. Naringine et Naringénine.
   Ces substances (flavanones) sont isolées et concentrées à patir d'un sirop commercial contenant, outre ces substances, un taux élevé de vitamine C. Cette dernière est éliminée et la préparation enrichie à 80-90 % en naringine (naringénine) selon la technique décrite par M. Beljanski dans le brevet français d'invention n° 88 08434 du 23 juin 1988.
III. R.L.B. : Les R.L.B. sont préparés à partir d'ARNS ribosomiques (E. Coli) découpés de façon standardisée (RNase pancréatique) selon la méthode qui se trouve décrite par M. Beljanski dans le brevet n° 76 16871 du 3 juin 1976 et Demande de certificat d'addition n° 7709184 du 28 mars 1977. Brevet USA 4 190 649, 1980.
   Ces ARN fragments ont été définis par leur taille, le rapport en bases puriques et bases pyrimidiques ainsi que par leur activité biologique qui est spécifique, dans les cellules du système hématopoietique.
IV. Bioparyl : extrait standardisé obtenu par différentes étapes de purification des feuilles dorées du Gingko Biloba. La méthode de préparation est décrite par M. Beljanski en détail dans le brevet français n° 88 09738 du 19.07.1988.

En conclusion, les subtances I et II sont nécessaires pour empêcher la multiplication virale (inhibition des transcriptions inverses) d'ADN polymerase et de la Désoxynucléotidyl transférase terminale (TdT) autant d'enzymes qui permettent la transcription, replication, l'intégration de l'ADN viral dans le génome de l'hôte et la multiplication du virus ; outre leur effet antiviral, ces substances possèdent un pouvoir anticancéreux qui permet de lutter contre les cellules cancéreuses provoquées ou non par les infections virales au-delà même de la barrière cérébrale.

Le mode d'action des substances I et II a été décrit par M. Beljanski dans les brevets français n° 88 08434 du 23 juin 1988 et n° 88 09738 du 19 juillet 1988.

Les R.L.B. (substance 3) assurent une protection et une génèse des plaquettes et des leucocytes. En outre, une très substantielle impulsion est donnée à la génèse des sous-populations lymphocytaires, phénomène d'importance essentielle dans la pathologie du Sida.

Enfin le Bioparyl régule les enzymes et protéines diverses s'accumulant dans l'organisme et participant à la pathologie.

## Revendications

1. Une composition pharmaceutique comportant :
(1) Flavopéreirine et/ou son dérivé dihydroflavopéreirine,
(2) Un ou plusieurs composés sélectionnés du groupe constitué par naringine, naringénine et des esters ou sels de ces derniers acceptables sur le plan pharmaceutique,
(3) ARN amorçeurs,
(4) Extrait standardisé obtenu à partir des feuilles dorées de l'arbre Gingko Biloba.

2. Une composition pharmaceutique selon la revendication 1, contenant un excipient pharmaceutique acceptable.

3. Une composition pharmaceutique selon la revendication 1 ou la revendication 2, présentée sous forme d'unité de dosage comprenant environ 0,5 à 1 gramme de (1), environ 0,5 à 1,5 grammes de (2), environ 10 à 40 milligrammes de (3) et de 0,5 à 2 grammes de (4).

4. Un kit de produits destiné au traitement des patients atteints de Sida, contenant des quantités individuellement empaquetées de (1) (2) (3) et (4) selon la revendication 1.

5. Utilisation d'une composition selon la revendication 1, pour l'obtention d'un médicament destiné au traitement des mammifères souffrant de maladie virale.

6. Utilisation selon la revendication 5, lorsque le mammifère est un être humain.

7. Utilisation selon la revendication 6, lorsque l'être humain est infecté par le virus du Sida.

8. Utilisation selon la revendication 7, lorsque l'être humain est infecté par des virus à ADN ou des virus à ARN.

9. Utilisation selon la revendication 7, lorsque l'être humain souffre d'un cancer.

10. Utilisation selon la revendication 5, pour l'obtention d'un médicament pour traiter un mammifère séropositif à l'infection par HIV, mais essentiellement sans symptômes de Sida.

11. Utilisation selon la revendication 6, dans laquelle on administre des doses quotidiennes de 0,25 à 1 gramme de flavopéreirine, de 0,5 à 1,5 grammes de naringine (naringénine), de 0,5 à 1 gramme de Bioparyl et de 10 à 40 milligrammes de R.L.B.

12. Utilisation selon la revendication 11, dans laquelle on réserve une dose journalière pour les R.L.B. et des prises fractionnées à plusieurs moments de la journée pour les autres produits.

13. Utilisation selon la revendication 11 ou la revendication 12, dans laquelle le R.L.B. est destiné à une administration sublinguale.

## Claims

1. A pharmaceutical composition comprising:
(1) flavopereirine and/or its derivative dihydroflavopereirine;
(2) one or more compounds selected from the group constituted by naringin, naringenin, and pharmaceutically acceptable esters and salts thereof;
(3) initiator RNA; and
(4) standardized extract obtained from the yellow leaves of the tree Gingko Biloba.

2. A pharmaceutical composition according to claim 1, containing a pharmaceutically acceptable vehicle.

3. A pharmaceutical composition according to claim 1, presented as a dosage unit comprising about 0.5 grams (g) to 1 g of (1), about 0.5 g to 1.5 g of (2), about 10 mg to about 40 mg of (3), and 0.5 g to 2 g of (4).

4. A kit of substances for use in treating patients suffering from AIDS, the kit containing individually packaged quantities of (1), (2), (3) and (4) according to claim 1.

5. Utilization of a composition according to claim 1, for obtaining a medicine for treating mammals suffering from a viral disease.

6. Utilization according to claim 5, when the mammal is a human being.

7. Utilization according to claim 6, when the human is infected with the human immunodeficiency virus.

8. Utilization according to claim 7, when the human is infected with DNA viruses or RNA viruses.

9. Utilization according to claim 7, when the human is suffering from a cancer.

10. Utilization according to claim 5, for obtaining a medicine for treating a person who is seropositive with HIV infection, but essentially without exhibiting the symptoms of AIDS.

11. Utilization according to claim 6, in which daily doses of 0.25 g to 1 g of flavopereirine, 0.5 g to 1.5 g of naringin (naringenin), and 0.5 g to 1 g of Bioparyl are administered while 10 mg to 40 mg of RLB are administered daily.

12. Utilization according to claim 11, in which RLB is administered once a day whereas the other substances are taken in fractions of a daily dose several times a day.

13. Utilization according to claim 11 or claim 12, in which RLB is intended for sublingual administration.

## Patentansprüche

1. Pharmazeutische Zusammensetzung, die enthält:
(1) Flavopereirin und/oder sein Derivat Dihydroflavopereirin,
(2) eine oder mehrere Verbindungen, die aus der Gruppe ausgewählt werden, die besteht aus Naringin, Naringenin und Estern oder Salzen dieser letzteren, die pharmazeutisch akzeptabel sind,
(3) auslösende RNS,
(4) einen genormten Extrakt, der aus den goldgelben Blättern des Gingko Biloba Baums hergestellt wird.

2. Pharmazeutische Zusamensetzung nach Anspruch 1, die eine akzetable pharmazeutische Grundmasse enthält.

3. Pharmazeutische Zusammensetzung nach Anspruch 1 oder Anspruch 2 in Form einer Dosiereinheit, die etwa 0,5 bis 1 Gramm von (1), etwa 0,5 bis 1,5 Gramm von (2), etwa 10 bis 40 Milligramm von (3) und 0,5 bis 2 Gramm von (4) enthält.

4. Baukasten von Präparaten zur Behandlung von AIDS-Kranken, der individuell verpackte Mengen von (1) (2) (3) und (4) gemäß Anspruch 1 enthält.

5. Verwendung einer Zusammensetzung nach Anspruch 1 zur Herstellung eines Medikaments, das zur Behandlung von Säugetieren bestimmt ist, die an einer Viruskrankheit leiden.

6. Verwendung nach Anspruch 5, wenn das Säugetier ein Mensch ist.

7. Verwendung nach Anspruch 6, wenn der Mensch mit dem AIDS-Virus infiziert ist.

8. Verwendung nach Anspruch 7, wenn der Mensch mit DMS enthaltenden Viren oder RNS enthaltenden Viren infiziert ist.

9. Verwendung nach Anspruch 7, wenn der Mensch an einem Krebs leidet.

10. Verwendung nach Anspruch 5 zur Herstellung eines Medikaments zur Behandlung eines Säugetiers, das in Bezug auf eine HIV-Infektion seropositiv, aber im wesentlichen ohne AIDS-Symptome ist.

11. Verwendung nach Anspruch 6, bei der man tägliche Dosen von 0,25 bis 1 Gramm von Flavopereirin, 0,5 bis 1,5 Gramm von Naringin (Naringenin), 0,5 bis 1 Gramm Bioparyl und 10 bis 40 Milligramm R.L.B. verabreicht.

12. Verwendung nach Anspruch 11, bei der man eine tägliche Dosis R.L.B. und zu mehreren Zeitpunkten des Tages Teildosen der anderen Produkte verabreicht.

13. Verwendung nach Anspruch 11 oder Anspruch 12, bei der R.L.B. sublingual verabreicht wird.
